(19) Europäisches Patentamt
European Patent Office
Office européen des brevets

(11) Numéro de publication: **0 188 947**
**B1**

(12) # FASCICULE DE BREVET EUROPEEN

(45) Date de publication du fascicule du brevet: **02.08.89**

(51) Int. Cl.⁴: **C07K 5/00**, C07K 5/02, A61K 37/02

(21) Numéro de dépôt: **85402527.7**

(22) Date de dépôt: **18.12.85**

(54) **Peptides réduits, inhibiteurs de la sécrétion gastrique, procédé d'obtention et compositions pharmaceutiques les contenant.**

(30) Priorité: **20.12.84 FR 8419544**

(43) Date de publication de la demande: **30.07.86 Bulletin 86/31**

(45) Mention de la délivrance du brevet: **02.08.89 Bulletin 89/31**

(84) Etats contractants désignés: **AT BE CH DE FR GB IT LI LU NL SE**

(56) Documents cités:
**FR-A-2 533 210**
**GB-A-1 042 481**

(73) Titulaire: **SANOFI S.A., 40, Avenue George V, F-75008 Paris(FR)**
Titulaire: **Etablissement Public dit: CENTRE NATIONAL DE LA RECHERCHE SCIENTIFIQUE (CNRS), 15, Quai Anatole France, F-75007 Paris(FR)**

(72) Inventeur: **Martinez, Jean, Rue des Cevennes, F-34720 Caux(FR)**
Inventeur: **Bali, Jean Pierre, 278, rue du Bosquet, F-34980 Saint Gely du Fesc(FR)**
Inventeur: **Magous, Richard, 318 Bd du Général de Gaulle, F-34400 Lunet(FR)**
Inventeur: **Castro, Bertrand, l'Estaple Avenue des Costières, F-34130 Saint Aunes(FR)**
Inventeur: **Demarne, Henri, Le Florence Avenue Major Flandre, F-34000 Montpellier(FR)**

(74) Mandataire: **Gillard, Marie-Louise, Cabinet Beau de Loménie 55, Rue d'Amsterdam, F-75008 Paris(FR)**

**EP 0 188 947 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux peptides inhibiteurs de la sécrétion gastrique. Elle concerne également un procédé pour leur obtention et les compositions pharmaceutiques les contenant.

La gastrine est une hormone gastro-instestinale qui possède,à un degré élevé,le pouvoir de stimuler la sécrétion gastrique.

Par ailleurs, la pentagastrine et la tétragastrine sont des peptides synthétiques proches de la séquence C-terminale des 5 ou 4 derniers aminoacides de la gastrine et répondent respectivement aux formules

$$Boc\text{-}bêta\text{-}Ala\text{-}Trp\text{-}Met\text{-}Asp\text{-}Phe\text{-}NH_2$$

$$\text{et} \quad H\text{-}Trp\text{-}Met\text{-}Asp\text{-}Phe\text{-}NH_2$$

utilisant, pour désigner les alpha-aminoacides et les groupes protecteurs, les abréviations à 3 lettres recommandées par la Commission de Nomenclature de l'IUPAC-IUB.

Ces composés sont également des stimulants de la sécrétion gastrique.

Selon la présente invention, il a été trouvé que, de façon surprenante, des dérivés peptidiques, dérivés de ces séquences par remplacement de la liaison amide -CO-NH- entre la méthionine et l'acide aspartique par une liaison réduite $-CH_2-NH-$ et remplacement éventuel de la méthionine par la leucine ou la norleucine, deviennent de puissants inhibiteurs de la sécrétion gastrique.

Les composés selon l'invention répondent à la formule générale :

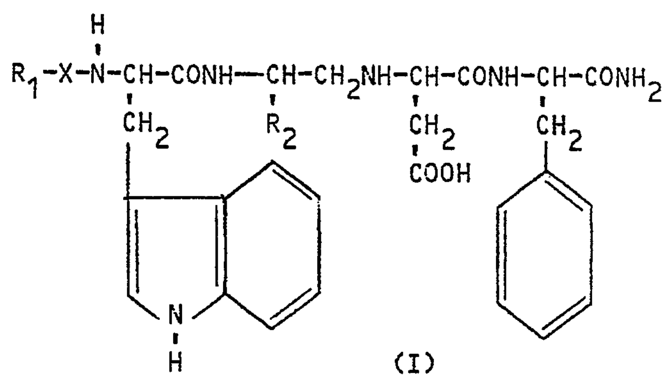

(I)

dans laquelle :

-$R_1$ représente l'hydrogène ou un groupe protecteur de la fonction amine, tel que t-butyloxy-carbonyle, benzyloxycarbonyle ou alcanoyle inférieur ;

-X représente la bêta-alanine, la glycine ou une liaison directe entre $R_1$ et le groupe amine,

-$R_2$ représente un groupe choisi parmi :

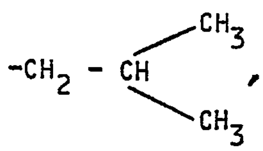 , $-CH_2-CH_2-S-CH_3$ ou $-(CH_2)_3-CH_3$ ;

correspondant aux chaînes latérales des aminoacides naturels : leucine, méthionine, norleucine.

Les sels que peuvent donner la fonction carboxylique de l'acide aspartique avec les bases minérales ou organiques font partie intégrante de l'invention.

La présente invention comprend également un procédé de préparation des composés de formule (I).

Le remplacement, dans une séquence peptidique, d'une liaison amide par une liaison $-CH_2-NH-$ peut être obtenu par action d'un alpha-aminoaldéhyde convenablement substitué sur un alpha-aminoacide en présence d'un agent de réduction tel qu'un borohydrure.

La réaction peut être schématisée par :

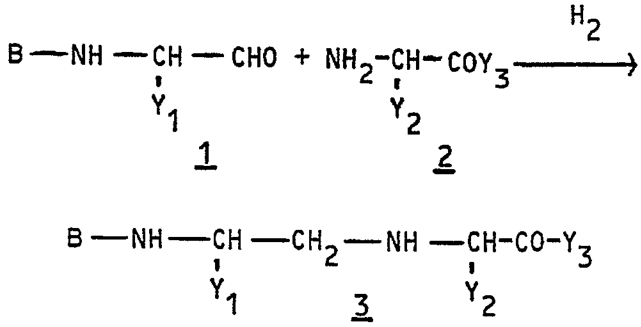


1 2

3

B représente un groupe protecteur de la fonction amine et notamment un groupe tertiobutoxycarbonyle ou un peptide protégé sur l'amine N-terminal, $Y_1$ et $Y_2$ représentent les chaînes latérales correspondant aux alpha-aminoacides naturels : leucine, norleucine, méthionine et acide aspartique ; $Y_3$ représente OH ou le résidu du phénylalaninamide.

L'imine qui se forme intermédiairement est réduite au fur et à mesure par action de l'hydrure utilisé, de préférence le cyanoborohydrure de sodium. On opère dans un solvant convenable, le plus souvent un alcool et on opère à une température comprise entre 20 et 50°C.

Lorsque les produits de départ comportent dans leurs chaînes latérales des substituants susceptibles de réagir avec l'aldéhyde ou avec le borohydrure, il convient de les bloquer avant réaction.

Ainsi, les groupes carboxyliques seront bloqués sous forme d'ester, de préférence d'ester benzylique.

Lorsque B est un groupe protecteur et/ou $Y_3$ est OH, l'élongation du peptide réduit peut être ensuite poursuivie par les méthodes habituelles de la chimie peptidique.

Les composés aldéhydiques 1 sont eux-mêmes préparés à partir des alpha-aminoacides correspondants. Toutefois, ces composés sont des composés chiraux et il convient de conserver pour l'aldéhyde l'isomérie optique du produit de départ. Pour ce faire, on pourra utiliser la méthode décrite dans le brevet français n° 2 531 078. Cette méthode peut être représentée par le schéma :

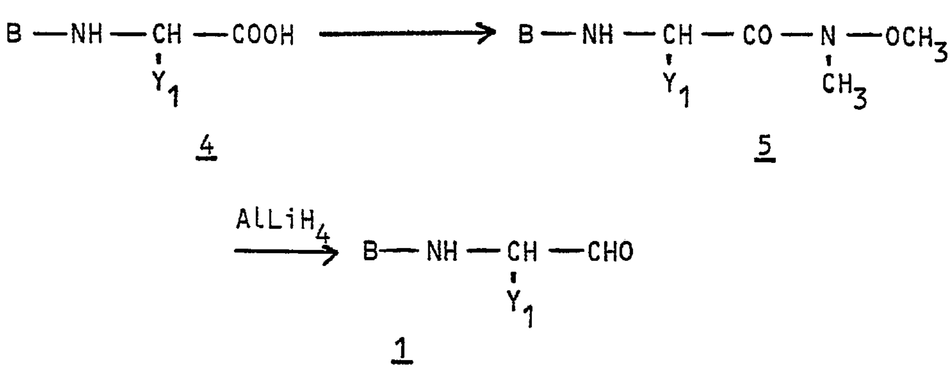


4 5

1

A partir de l'acide 4, l'action de la N,O-diméthyl-hydroxylamine conduit au N,O-diméthylhydroxamate 5. Celui-ci, par réduction par l'hydrure de lithium-aluminium, conduit à l'aldéhyde 1 qui conserve la stéréochimie de l'acide 4.

Les fragments des composés (I), qui sont des fragments peptidiques, peuvent être préparés selon les méthodes habituelles de synthèse peptidique en phase liquide. A partir de l'aminoacide C-terminal, on introduit successivement les aminoacides présents dans la séquence.

Les réactions de couplage sont effectuées soit avec un ester activé de l'acide aminé à introduire au sein du diméthyl-formamide et en présence de diisopropyléthylamine et d'hydroxy-1 benzotriazole, soit avec l'acide aminé au sein du diméthylformamide en présence d'hexafluorophosphate de benzotriazolyloxy-tris-(diméthylamino) phosphonium et de diisopropyléthylamine.

Tous les aminoacides sont incorporés sous la forme du dérivé protégé sur l'amine en alpha, le groupe protecteur choisi étant le groupe t-butyloxycarbonyle. Lorsque l'aminoacide utilisé présente dans sa chaîne latérale des fonctions susceptibles de réagir, celles-ci doivent être bloquées au préalable. Ainsi, les fonctions acides en bêta de l'acide aspartique doivent être bloquées sous forme d'ester, en particulier l'ester benzylique.

Après chaque réaction de couplage, la déprotection de l'amine en alpha est effectuée par hydrolyse acide.

Finalement, les produits, protégés sur les fonctions figurant dans leurs chaînes latérales,sont déprotégés pour conduire aux composés de formule (I).

Les exemples suivants permettront de mieux comprendre l'invention.

Dans ces exemples, on utilisera les abréviations suivantes :

Acides aminés et groupes protecteurs :

Gly : Glycine
Phe : L-phénylalanine
Asp : L-acide aspartique
Leu : L-leucine
Met : L-méthionine
Nle : L-norleucine
OBzl : ester benzylique
Boc : t-butyloxycarbonyle
Trp : Tryptophane

ONp : 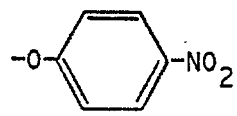

OBut : Ester tertiobutylique
OSu : Ester de N-hydroxy succinimide

Autres abréviations :

TFA : acide trifluoracétique
DMF : diméthylformamide
DIEA : diisopropyléthylamine

BOP : 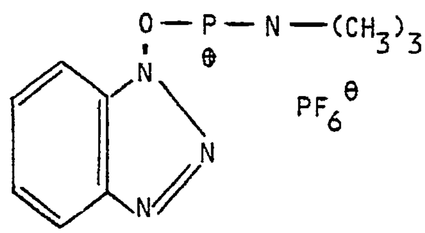

HOBt : hydroxy-1 benzotriazole

Exemple 1

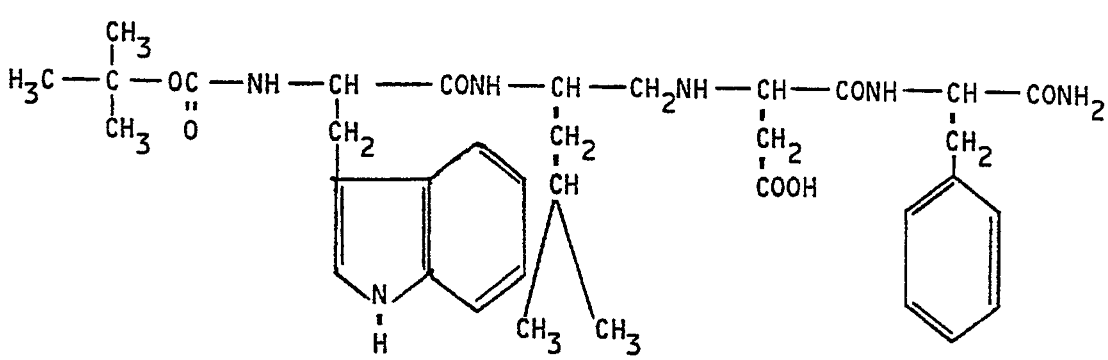

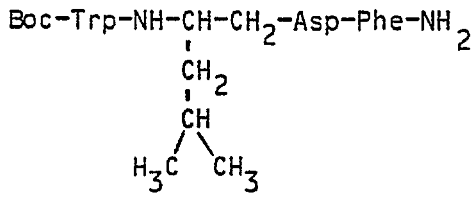

A) Boc–Asp(bëta OBzl)–Phe–$NH_2$

On dissout 2,88 g de phénylalaninamide, 6,63 g de BOP et 4,85 g de Boc-Asp (bêta OBzl) dans 20 ml de DMF. On ajoute 3 ml de DIEA et laisse 10 h sous agitation à température ambiante. On évapore le DMF sous vide à température inférieure à 40°C. On dissout le résidu dans 250 ml d'acétate d'éthyle et lave la solution 2 fois avec 50 ml de solution à 10 % d'acide citrique et 1 fois avec 100 ml d'eau. On sèche sur sulfate de sodium et concentre sous vide.

Le résidu cristallise par trituration avec de l'éther : F = 86-89°C ; (alpha)D = -22,5° (c = 1,1 DMF) ; Rendement 6,5 g soit 92 %.

B) 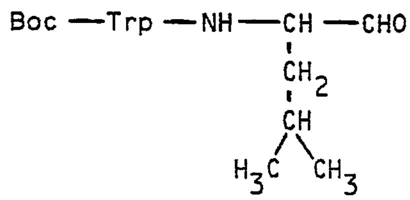

1) 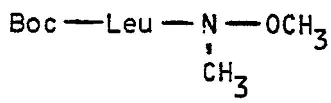

On dissout 4,98 g de Boc-Leu monohydrate, 8,84 g de BOP et 2,1 g de chlorhydrate de N,O-diméthylhydroxylamine dans 150 ml de dichlorométhane. On ajoute 6,9 ml de DIEA et laisse 5 h à température ambiante. On évapore le solvant sous vide et reprend le résidu dans l'acétate d'éthyle. On lave la solution 2 fois avec une solution saturée de bicarbonate de sodium, 2 fois avec une solution à 10 % d'acide citrique et 1 fois avec de l'eau. On sèche la solution sur sulfate de sodium et évapore sous vide.

On chromatographie le résidu sur une colonne de gel de silice. En éluant avec un mélange acétate d'éthyle-hexane 1/1 vol/vol, on obtient une huile incolore (4,5 g) : Rendement : 82 %.

<u>Chromatographie sur couche mince :</u>

Rf = 0,6 (acétate d'éthyle-hexane 1/1 vol/vol).

2) 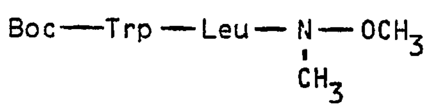

On traite 2,74 g du produit préparé ci-dessus par 10 ml de TFA pendant 30 min à température ambiante. On évapore le TFA en présence d'éther plusieurs fois sous vide. Le résidu huileux incolore est séché au dessiccateur sur potasse.

On dissout le trifluoracétate ainsi obtenu dans 20 ml de DMF avec 4,06 g de Boc-Trp-ONp, 1,4 g d'HOBt et 3,75 ml de DIEA. On agite pendant 10 h à température ambiante puis on évapore le solvant sous vide à température inférieure à 40°C. On dissout le résidu dans l'acétate d'éthyle et lave la solution, successivement, 2 fois avec une solution saturée de bicarbonate de sodium, 2 fois avec une solution d'acide citrique à 10 % et 1 fois avec de l'eau. On sèche sur sulfate de sodium et concentre sous vide. On chromatographie le résidu sur une colonne de gel de silice. En éluant avec le mélange acétate d'éthyle-hexane 1/1 vol/vol, on obtient une poudre incolore (3,5 g) : F = 75-80°C (décomposition ; rendement 85 %.

3) 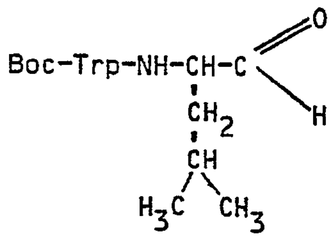 ou Boc-Trp-leucinal

On dissout 2,30 g du produit obtenu ci-dessus dans 10 ml de tétrahydrofuranne anhydre puis on ajoute 50 ml d'éther anhydre. On ajoute ensuite par fractions 0,76 g d'hydrure de lithium aluminium. Après la fin de l'addition, on laisse réagir 45 min, puis on verse lentement le mélange réactionnel dans 150 ml d'une solution refroidie d'acide citrique à 10 %. On laisse 40 min sous agitation puis on dilue avec 150 ml d'éther et décante la phase organique. On la lave successivement avec une solution d'acide citrique à 10 %, avec de l'eau, avec une solution saturée de bicarbonate de sodium et avec de l'eau. On sèche sur sulfate de sodium puis concentre sous vide à température inférieure à 40°C.

Il reste une huile incolore qui doit être utilisée rapidement pour la suite des opérations. On peut toutefois la conserver quelques heures au réfrigérateur.

C) 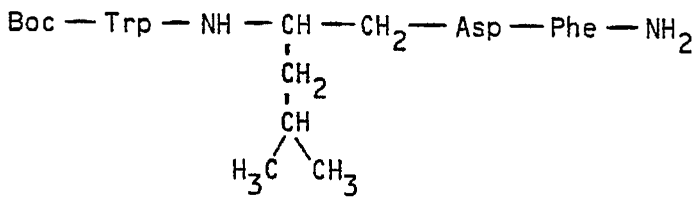

1) 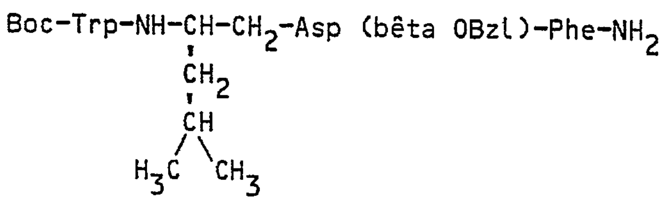

On traite 1,64 g du peptide protégé préparé au paragraphe A par 10 ml de TFA pendant 30 min à température ambiante. On ajoute 150 ml d'éther et essore le précipité qui s'est formé. On le lave plusieurs fois avec de l'éther puis on le sèche sous vide.

On dissout le trifluoracétate ainsi obtenu dans 100 ml de méthanol avec l'aldéhyde obtenu au paragraphe B-3, provenant de la réduction de 2,3 g de N,O-diméthylhydroxamate.

On amène le pH à 9 par addition de DIEA. On ajoute une solution de 0,225 g de cyanoborohydrure de sodium dans 2 ml de méthanol et ajuste le pH à 6 par addition d'une solution aqueuse de bisulfate de potassium à 10 %. Pendant toute la durée de l'opération, le pH est maintenu entre 6 et 6,5 par addition de solution de bisulfate de potassium à 10 %.

Après 1 h, on ajoute à nouveau une solution de 0,225 g de cyanoborohydrure de sodium dans 2 ml de méthanol et, après 6 h, on effectue une nouvelle addition (même quantité).

Après 20 h, le pH n'évolue plus. On concentre sous vide à température inférieure à 40°C. On reprend le résidu dans l'acétate d'éthyle et on lave la solution avec une solution saturée de bicarbonate de sodium puis avec de l'eau. On sèche sur sulfate de sodium puis concentre sous vide. Le résidu est chromatographié sur une colonne de gel de silice. On élue avec le mélange acétate d'éthyle-hexane 7/3 vol/vol. En triturant le produit obtenu avec de l'éther, on obtient une poudre incolore (0,43 g) : F = 199-201°C ; (alpha)D = -10,5° (c = 0,2 DMF).

Selon une variante du procédé, on peut opérer à chaud. On opère sur les mêmes quantités de réactif dissoutes dans 20 ml de méthanol contenant 0,3 ml d'acide acétique. Après 30 min d'agitation, on ajoute par fractions en 35 min la solution de 0,65 g de cyanoborohydrure de sodium dans 5 ml de méthanol. On chauffe ensuite à 40°C pendant 2 h puis traite comme indiqué ci-dessus. Après chromatographie sur gel de silice, on obtient 0,52 g de produit identique à celui obtenu précédemment.

2) 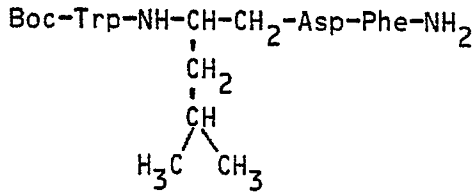

On dissout 0,3 g du composé obtenu au paragraphe 1 ci-dessus dans 20 ml d'éthanol à 95° et hydrogène à température et pression ordinaires en présence de 0,03 g de palladium sur charbon à 10 %. Après 3 h, on filtre le catalyseur et évapore le solvant sous vide à température inférieure à 40°C. Le résidu est trituré avec de l'éther. On essore le solide et lave plusieurs fois avec de l'éther. On obtient une poudre incolore (0,21 g) : F = 180°C (décomposition) ; (alpha)D = -20°C (c = 0,32 DMF).

Exemple 2

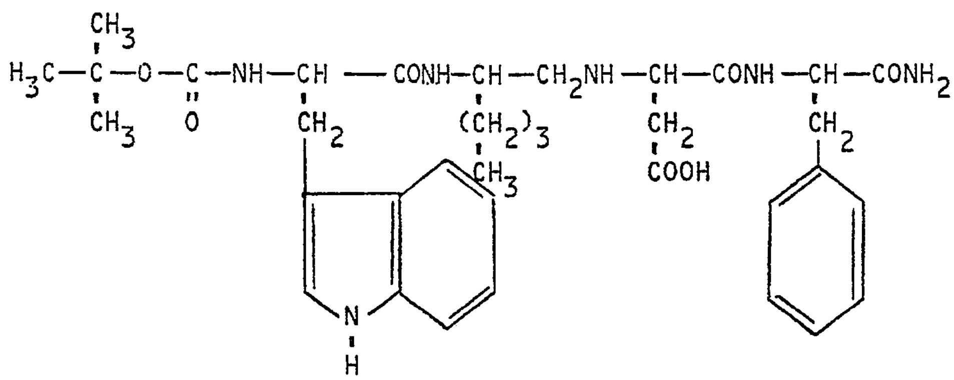

ou

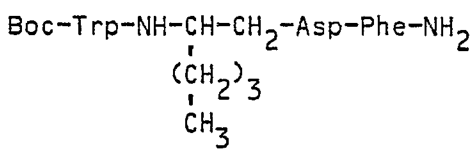

1) 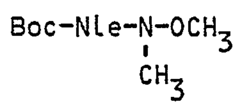

On opère comme dans l'exemple 1B-1 en remplaçant la leucine par la norleucine. On isole le produit attendu sous forme d'une huile.

Rendement: 84%; Rf = 0,68 (acétate d'éthyle-hexane 1/1 vol/vol).

2) 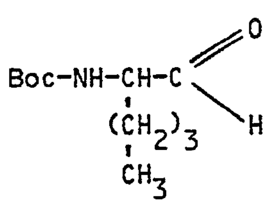 ou Boc-Norleucinal

A la solution refroidie à 0°C de 1,9 g de l'hydroxamate obtenu ci-dessus dans 50 ml d'éther, on ajoute par portions, en 15 min, 0,79 g d'hydrure de lithium aluminium.

Après la fin de l'addition, on laisse 15 min sous agitation puis on ajoute 50 ml d'acétate d'éthyle puis 100 ml d'une solution aqueuse froide à 10 % d'acide citrique. On agite vigoureusement pendant 30 min puis on sépare la phase organique qu'on lave avec 50 ml d'une solution d'acide citrique à 10 % puis avec de l'eau.

On sèche la solution sur sulfate de magnésium puis évapore le solvant sous vide. Il reste une huile utilisée telle quelle dans l'étape suivante.

Rf = 0,86 (acétate d'éthyle-hexane 1/1 vol/vol).

3) Boc-NH-CH-$CH_2$-Asp (bêta OBzl)-Phe-$NH_2$
$(CH_2)_3$
$CH_3$

On déprotège 2 g du dipeptide protégé obtenu à l'exemple 1A par action de 5 ml de TFA pendant 30 min. Par addition d'éther, il se sépare un solide incolore qu'on essore, lave plusieurs fois avec de l'éther puis sèche sous vide sur potasse.

Ce solide est dissous dans la solution du Boc-Norleucinal préparé ci-dessus dans 30 ml de méthanol contenant 1 % d'acide acétique.

On ajoute ensuite à température ambiante, par portions, en 30 min, 0,8 g de cyanoborohydrure de sodium. Après l'addition, on laisse 30 min de plus à température ambiante puis évapore le solvant sous vide et on traite le résidu avec 50 ml d'une solution saturée de bicarbonate de sodium. On extrait 3 fois avec de l'acétate d'éthyle (30 ml) et réunit les extraits qu'on lave avec de l'eau puis sèche sur sulfate de magnésium.

Par addition de 150 ml d'hexane, il se sépare un solide qu'on essore, lave avec de l'hexane et sèche sous vide.

Poids = 1,87 g; Rendement: 78%; F = 177–179°C (décomposition).

(alpha)D = –14,7° (c = 1,33 DMF).

4) Boc-Trp-NH-CH-$CH_2$-Asp (bêta OBzl)-Phe-$NH_2$
$(CH_2)_3$
$CH_3$

On déprotège 1,7 g du peptide ci-dessus par 5 ml de TFA comme indiqué au paragraphe 3.

Le solide ainsi obtenu est dissous dans 10 ml de DMF. On ajoute 1,04 g de Boc-Trp-OSu et 1,03 ml de DIEA. On agite le mélange pendant 3 h à température ambiante. On ajoute une solution d'acide citrique à 5 % et essore le solide précipité. On le lave avec de l'eau, une solution aqueuse de bicarbonate de sodium et à nouveau avec de l'eau. On sèche le solide sous vide sur anhydride phosphorique. On obtient 1,3 g du solide sec.

Rendement: 66%; F = 196–198°C (décomposition); (alpha)D = –9,7° (c = 1,2 DMF).

5) Boc-Trp-NHCH-$CH_2$-Asp-Phe-$NH_2$
$(CH_2)_3$
$CH_3$

On soumet 1 g du produit obtenu au paragraphe 4 ci-dessus à une hydrogénation catalytique en présence de palladium sur charbon à 10 % suivant la méthode indiquée à l'exemple 1C-2.

De la même façon, on isole 0,87 g du produit attendu.

Rendement: 99 % ; F = 122–124°C (décomposition); (alpha)D = –17,5° (c = 0,7 DMF).

Sel d'ammonium.

On dissout ce composé dans une solution 0,1 N d'ammoniaque, filtre la solution sur millipore et lyophilise.

Exemple 3

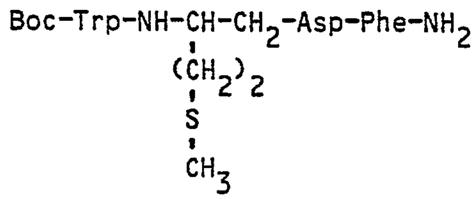

On opère comme dans l'exemple 2 en remplaçant la L-Norleucine par la L-méthionine. Dans ce cas, le groupe carboxylique de la chaîne latérale de l'acide aspartique a été protégé sous forme d'ester tertiobutylique au lieu d'ester benzylique. Ce groupe protecteur s'élimine en milieu acide fort en même temps que le groupe amino protecteur Boc avant la condensation du tryptophane.

On a obtenu successivement :

– Boc-Met-N,0-diméthylhydroxamate, Huile, Rf 0,42 (acétate d'éthyle-hexane 1/1 vol/vol).

– Boc-méthioninal, Huile, Rf 0,55 (acétate d'éthyle-hexane 1/1 vol/vol).

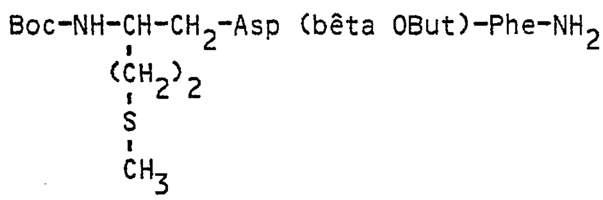

F = 118–120°C (acétate d'éthyle-hexane 1/1 vol/vol)
(alpha)D = –7,4° (c = 1,1 DMF).

–

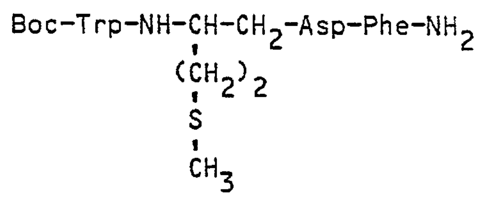

F = 189–190°C (chromatographie sur colonne de silice, éluant : acétate d'éthyle, pyridine, acide acétique, eau 80/20/5/10 vol/vol).
(alpha)D = –24,2° (c = 1,2 DMF).

Exemple 4

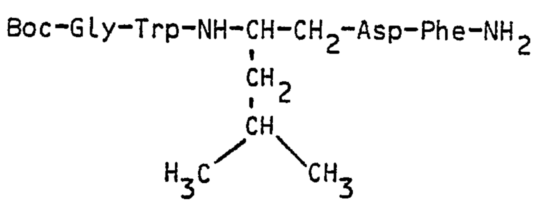

Ce composé a été obtenu à partir du composé de l'exemple 1 qu'on déprotège par action de TFA comme indiqué précédemment et sur lequel on condense un ester activé de la Boc-glycine.
F = 125°C (décomposition)
(alpha)D = -13,3° (c = 0,68 DMF).

Les composés selon l'invention ont été étudiés en ce qui concerne leurs propriétés thérapeutiques. Plus particulièrement, ces composés ont été étudiés in vivo en ce qui concerne leur effet sécrétoire gastrique chez le rat.

Le modèle choisi pour la mesure de l'effet sécrétoire est celui de l'estomac de rat anesthésié reperfusé. Le protocole suivi est une modification de celui décrit préalablement par Ghosh et Schild.

Un rat mâle de souche Wistar de 300 g, à jeun de 18 h, est anesthésié à l'uréthane (solution à 10 %, 1,5 ml/100 g i.p.). On pratique ensuite une trachéotomie et un cathétérisme de la veine du pénis qui permettra l'administration i.v. des peptides. On place ensuite une canule dans l'oesophage jusqu'au cardia et une seconde dans le duodénum (par une duodénotomie effectuée à 3 cm environ du pylore) jusque dans la zone antrale gastrique.

Un soluté propionique-succinique (pH 5,5) qui donne une variation linéaire du pH en fonction de la concentration en ions $H^+$ est utilisé pour perfuser l'estomac en circuit ouvert ou fermé avec un débit de 3 ml/min. La température corporelle, ainsi que celle du soluté,est contrôlée et maintenue à 30°C. La sécrétion acide de l'estomac va entraîner une variation du pH détectée par l'électrode de verre et enregistrée en fonction du temps.

Après stabilisation de la sécrétion basale, on injecte la gastrine par voie intraveineuse soit en perfusion, soit en injection unique. La réponse est enregistrée en fonction du temps et la quantité d'acide sécrété est mesurée sur l'enregistrement par différence avec la sécrétion basale.

La même expérience est réalisée soit en injectant le peptide à étudier i.v. sur le plateau de sécrétion acide, soit en associant le peptide avec le stimulant dans des rapports de concentration variables. Enfin, le peptide est administré seul à différentes doses pour examiner son effet agoniste.

Les expériences réalisées avec le composé de l'exemple 1 ont donné les résultats suivants :

Effet agoniste

Le produit de l'exemple 1 n'a montré aucun effet agoniste jusqu'à la dose de 1 mg/kg.

Effet antagoniste

Le composé de l'exemple 1 a été étudié à différentes doses en ce qui concerne son activité antagoniste.

Les résultats obtenus ont permis de déterminer la dose efficace 50 (DE 50) ou dose qui inhibe de 50 % la sécrétion gastrique stimulée par la gastrine.

Pour le composé de l'exemple 1, la DE 50 est de 0,3 mg/kg.

De la même façon, les composés des exemples 2 et 3 étudiés de manière analogue n'ont pas montré d'effet agoniste. Leur effet antagoniste s'est manifesté pour une dose efficace 50 de 0,3 mg/kg et 0,5 mg/kg respectivement pour les composés des exemples 2 et 3.

D'après ces résultats, on constate que :

- Les composés selon l'invention présentent un effet agoniste pratiquement nul vis-à-vis de la gastrine et ce malgré la valeur élevée des doses utilisées.
- Les composés selon l'invention présentent un effet inhibiteur important sur la sécrétion gastrique dans les conditions expérimentales utilisées.
- Par ailleurs, ces composés sont peu toxiques.

Par suite, les composés selon l'invention pourront être utilisés en thérapeutique humaine dans tous les cas où il est utile de diminuer la sécrétion gastrique et en particulier pour le traitement des ulcères gastro-duodénaux.

Les composés de la présente invention peuvent être utilisés de préférence par voie injectable : intraveineuse, intramusculaire ou sous-cutanée. Ils sont utilisés dans un solvant tel que le sérum physiologique (solution saline isotonique).

La présente invention concerne donc également les compositions pharmaceutiques contenant, à titre d'ingrédient actif, un peptide selon l'invention en combinaison avec un véhicule pharmaceutiquement acceptable, tel que le sérum physiologique.

La posologie peut varier suivant l'intensité de l'effet thérapeutique recherché, la gravité de l'affection à traiter et la voie d'administration utilisée. Elle doit donc être déterminée, pour chaque patient, en fonction de ces divers critères. Le plus souvent, elle est comprise entre 0,1 et 10 mg de principe actif par kg de poids corporel.

**Revendications pour les etats contractant, BE, CH, LI, DE, FR, GB, IT, LU, NL, SE**

1. A titre de produits nouveaux, les peptides répondent à la formule générale :

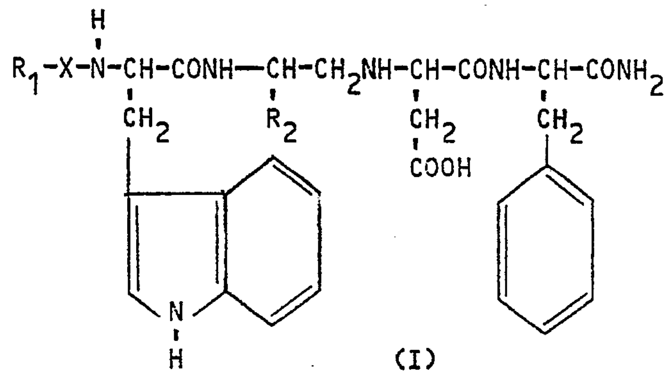

(I)

dans laquelle

- $R_1$ représente l'hydrogène ou un groupe protecteur de la fonction amine, tel que les groupes t-butyloxycarbonyle, benzyloxycarbonyle ou alcanoyle inférieur ;
- X représente la bêta-alanine, la glycine ou une liaison directe entre $R_1$ et le groupe amine ;
- $R_2$ représente un groupe choisi parmi :

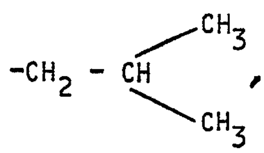
, $-CH_2-CH_2-S-CH_3$ ou $-(CH_2)_3-CH_3$ ;

correspondant aux chaînes latérales des aminoacides naturels : leucine, méthionine, norleucine, ainsi que leurs sels avec les bases minérales ou organiques.

2. Peptides selon la revendications 1, caractérisés en ce qu'ils répondent la formule :

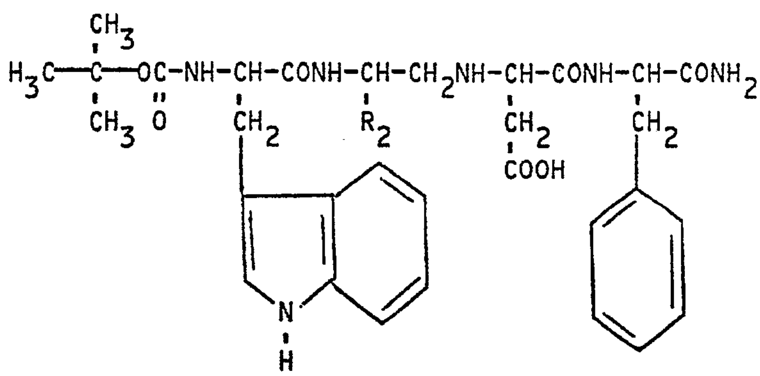

où $R_2$ a la signification mentionnée dans la revendication 1.

3. Peptides selon la revendication 1, caractérisés en ce que X est la glycine et $R_2$ est la chaine latérale de la leucine.

4. Procédé pour l'obtention des peptides selon l'une quelconque des revendications 1 à 3, caractérisé en ce qu'il consiste à faire réagir, en présence d'un agent de réduction, un alpha-aminoaldéhyde convenablement substitué de formule :

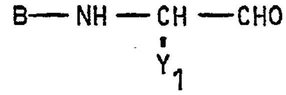

avec un alpha-aminoacide de formule:

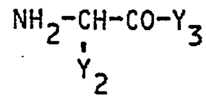

formules dans lesquelles : B représente un groupe protecteur de la fonction amine ou un peptide protégé sur l'amine N-terminal, $Y_1$ et $Y_2$ représentent les chaînes latérales correspondant aux alpha-aminoacides naturels : leucine, norleucine, méthionine et acide aspartique et $Y_3$ représente OH ou le résidu du phénylalaninamide et qu'éventuellement, si B est un groupe protecteur et/ou $Y_3$ est OH, on poursuit l'élongation du peptide réduit par les méthodes habituelles de la chimie peptidique.

5. Composition pharmaceutique inhibitrice de la sécrétion gastrique, caractérisée en ce qu'elle contient, à titre d'ingrédient actif, un peptide selon l'une quelconque des revendications 1 à 3, en association avec un véhicule pharmaceutiquement acceptable.

**Revendications pour l'Etat contractant : AT**

1. Procédé pour l'obtention des peptides répondant à la formule générale

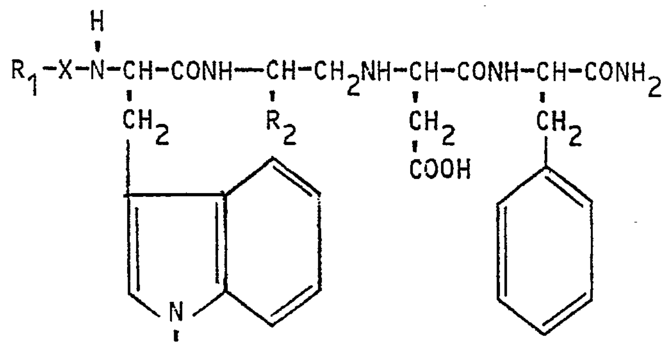

(I)

dans laquelle

- $R_1$ représente l'hydrogène ou un groupe protecteur de la fonction amine, tel que les groupes t-butyloxycarbonyle, benzyloxycarbonyle ou alcanoyle inférieur ;
- X représente la bêta-alanine, la glycine ou une liaison directe entre $R_1$ et le groupe amine ;
- $R_2$ représente un groupe choisi parmi :

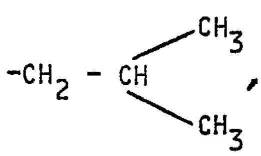 , $-CH_2-CH_2-S-CH_3$ ou $-(CH_2)_3-CH_3$ ;

correspondant aux chaînes latérales des aminoacides naturels : leucine, méthionine, norleucine, ainsi que leurs sels avec les bases minérales ou organiques, caractérisé en ce qu'il consiste à faire réagir, en présence d'un agent de réduction, un alpha-aminoaldéhyde convenablement substitué de formule

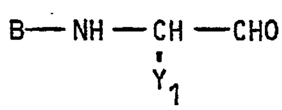

avec un alpha-aminoacide de formule:

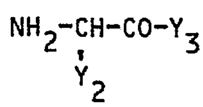

formules dans lesquelles: B représente un groupe protecteur de la fonction amine ou un peptide protégé sur l'amine N-terminal, $Y_1$ et $Y_2$ représentent les chaînes latérales correspondant aux alpha-aminoacides naturels : leucine, norleucine, méthionine et acide aspartique et $Y_3$ représente OH ou le résidu du phényl-alaninamide et qu'éventuellement, si B est un groupe protecteur et/ou $Y_3$ est OH, on poursuit l'élongation du peptide réduit par les méthodes habituelles de la chimie peptidique.

2. Procédé selon la revendication 1, caractérisé en que $R_1$ est Boc ; X est une liaison directe et $R_2$ est tel que défini dans la revendication 1.

3. Procédé selon la revendication 1, caractérisé en ce que X est la glycine, $R_1$ est Boc et $R_2$ est la chaine latérale de la leucine.

4. Utilisation des peptides obtenus selon l'une quelconque des revendications 1 à 3, pour la préparation de compositions pharmaceutiques contenant à titre d'ingrédient actif un peptide de formule I en combinaison avec un véhicule pharmaceutiquement acceptable, tel que le sérum physiologique.

**Patentansprüche für die Vertragsstaaten BE, CH, LI, DE, FR, GB, IT, LU, NL, SE**

1. Als neue Produkte die Peptide der allgemeinen Formel:

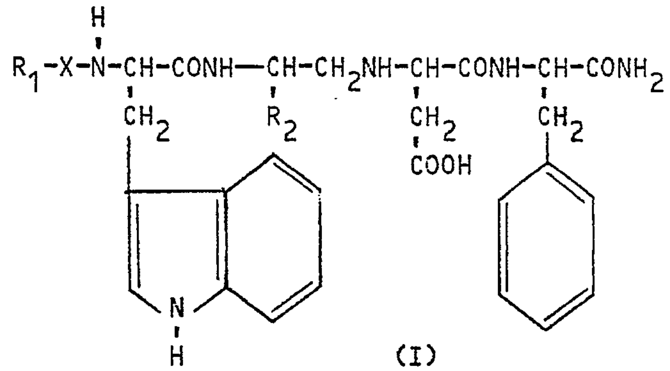

(I)

worin:
- $R_1$ Wasserstoff oder eine Schutzgruppe der Aminofunktion, wie die Gruppen tert. Butoxycarbonyl, Benzyloxycarbonyl oder Niedrigalkanoyl, darstellt;
- X Beta-Alanine, Glycin oder eine Direktbindung zwischen $R_1$ und der Aminogruppe darstellt;
- $R_2$ eine aus:

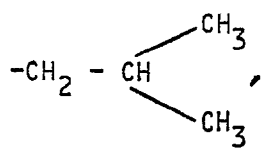 , $-CH_2-CH_2-S-CH_3$ ou $-(CH_2)_3-CH_3$ ;

gewählte Gruppe darstellt, entsprechend den Seitenketten der natürlichen Aminosäuren: Leucin, Methionin, Norleucin, sowie deren Salze mit den anorganischen oder organischen Basen.

2. Peptide nach Anspruch 1, dadurch gekennzeichnet, dass sie der Formel:

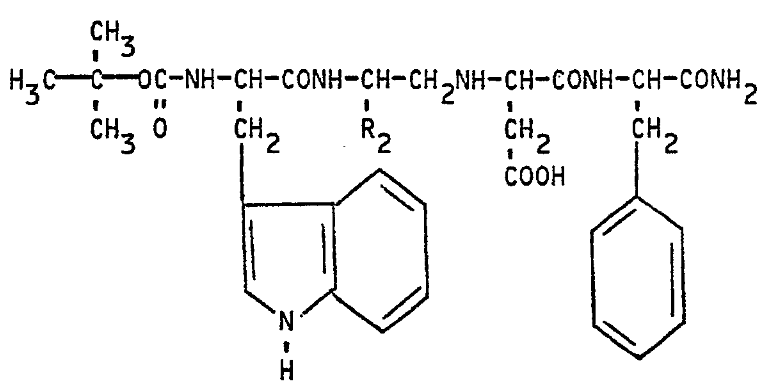

entsprechen, worin $R_2$ die in Anspruch 1 genannte Bedeutung hat.

3. Peptide nach Anspruch 1, dadurch gekennzeichnet, dass das X Glycin und $R_2$ die Seitenkette des Leucin ist.

4. Verfahren zur Herstellung der Peptide nach einem der Ansprüche 1 bis 3, dadurch gekennzeichnet, dass es darin besteht, dass in Gegenwart eines Reduktionsmittels, ein angemessen substituiertes Alpha-Aminoaldehyd der Formel:

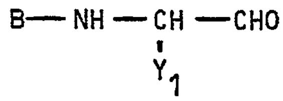

mit einer Alpha-Aminosäure der Formel:

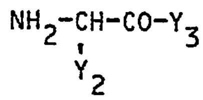

reagiert wird, in welchen Formeln: B eine Schutzgruppe der Aminofunktion oder ein auf dem N-terminalen Amin geschütztes Peptid darstellt,

$Y_1$ und $Y_2$ Seitenketten darstellen, die den natürlichen Alpha-Aminosäuren: Leucin, Norleucin, Methionin und Asparaginsäure entsprechen und

$Y_3$ OH oder den Phenylalaninamidrest darstellt und dass man gegebenfalls, wenn B eine Schutzgruppe und/oder $Y_3$ OH ist, die Elongation des reduzierten Peptids mit den in der Peptid-Chemie üblichen Methoden fortsetzt.

5. Pharmazeutische Zusammensetzung zur Inhibition der Magensaftsekretion, dadurch gekennzeichnet, dass sie als aktives Ingrediens ein Peptid nach einem der Ansprüche 1 bis 3 in Kombination mit einem pharmazeutischen akzeptablen Vehikel enthält.

**Patentansprüche für den Vertragsstaat AT**

1. Verfahren für die Herstellung der Peptide nach der allgemeinen Formel

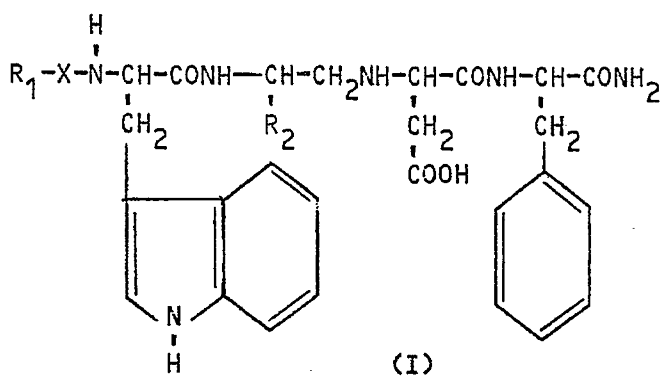

(I)

worin:

– $R_1$ Wasserstoff oder eine Schutzgruppe der Aminofunktion, wie die Gruppen tert. Butoxycarbonyl, Benzyloxycarbonyl oder Niedrigalkanoyl, darstellt;
– X Beta-Alanine, Glycin oder eine Direktbindung zwischen $R_1$ und der Aminogruppe darstellt;
– $R_2$ eine aus:

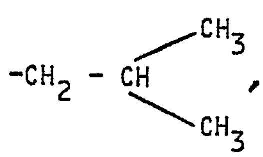 , $-CH_2-CH_2-S-CH_3$ ou $-(CH_2)_3-CH_3$ ;

gewählte Gruppe darstellt, entsprechend den Seitenketten der natürlichen Aminosäuren: Leucin, Methionin, Norleucin, sowie deren Salze mit den anorganischen oder organischen Basen, dadurch gekennzeichnet, dass es darin besteht, dass in Gegenwart eines Reduktionsmittels, ein angemessen substituiertes Alpha-Aminoaldehyd der Formel:

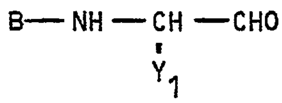

mit einer Alpha-Aminosäure der Formel:

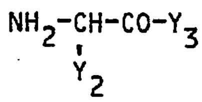

reagiert wird, in welchen Formeln: B eine Schutzgruppe der Aminofunktion oder ein auf dem N-terminalen Amin geschütztes Peptid darstellt,

$Y_1$ und $Y_2$ Seitenketten darstellen, die den natürlichen Alpha-Aminosäuren: Leucin, Norleucin, Methionin und Asparaginsäure entsprechen und

$Y_3$ OH oder den Phenylalaninamidrest darstellt und dass man gegebenfalls, wenn B eine Schutzgruppe und/oder $Y_3$ OH ist, die Elongation des reduzierten Peptids mit den in der Peptid-Chemie üblichen Methoden fortsetzt.

2. Verfahren nach Anspruch 1, dadurch gekennzeichnet, dass $R_1$ Boc ist; X eine Direktbindung ist und $R_2$ so, wie in Anspruch 1 definiert, ist.

3. Verfahren nach Anspruch 1, dadurch gekennzeichnet, dass X Glycin, $R_1$ Boc und $R_2$ die Seitenkette des Leucin ist.

4. Verwendung der nach einem der Ansprüche 1 bis 3 erhaltenen Peptide zur Herstellung von pharmazeutischen Zusammensetzungen, die als aktives Ingrediens ein Peptid der Formel I in Kombination mit einem pharmazeutisch akzeptablen Vehikel, wie das physiologische Serum enthalten.

**Claims for the contracting states: BE, CH, LI, DE, FR, GB, IT, LU, NL, SE**

1. As new products, the peptides correspond to the general formula:

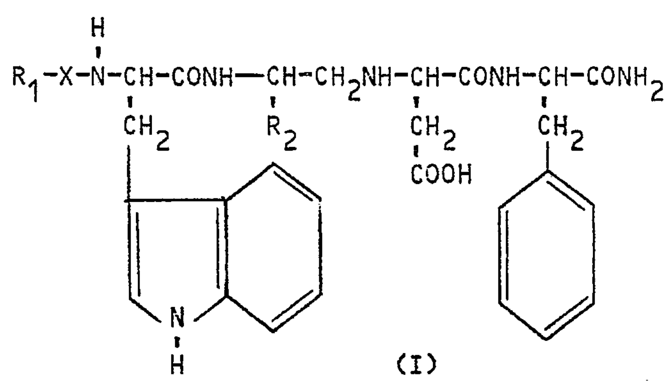

(I)

in which:

– $R_1$ represents hydrogen or a protecting group for the amine group, such as t-butoxycarbonyl, benzyloxycarbonyl or lower alkanoyl groups;

– X represents beta-alanine, glycine or a direct bond between $R_1$ and the amine group;

– $R_2$ represents a group chosen from:

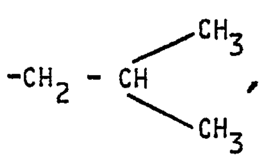 , $-CH_2-CH_2-S-CH_3$ ou $-(CH_2)_3-CH_3$ ;

corresponding to the side chains or the natural amino acids: leucine, methionine and norleucine, and also their salts with inorganic or organic bases.

2. The peptides according to claim 1, characterized in that they correspond to the formula:

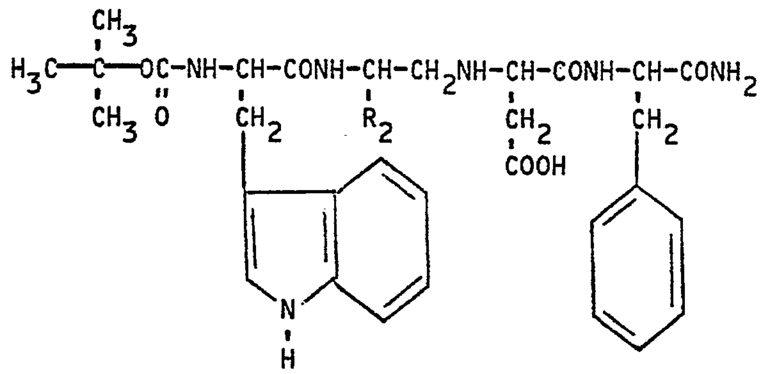

in which $R_2$ has the meaning mentioned in claim 1.

3. The peptides according to claim 1, characterized in that X is glycine and $R_2$ is the side chain of leucine.

4. A process for the preparation of the peptides according to any one of claims 1 to 3, characterized in that an appropriately substituted alpha-amino aldehyde of the formula:

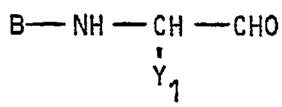

is reacted, in the presence of a reducing agent, with an alpha-amino acid of the formula:

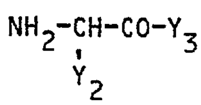

in which formulae: B represents a protecting group for the amine group or a peptide protected on the terminal N amine,

$Y_1$ and $Y_2$ represent the side chains corresponding to the natural alpha-amino acids: leucine, norleucine, methionine and aspartic acid, and

$Y_3$ represents OH or the residue of phenylalaninamide, and that, if appropriate, if B is a protecting group and/or $Y_3$ is OH, the reduced peptide is lengthened by the methods usually employed in peptide chemistry.

5. A pharmaceutical composition for inhibiting gastric secretion, characterized in that it contains a peptide according to any one of claims 1 to 3 as the active ingredient, in association with a pharmaceutically acceptable vehicle.

**Claims for the contracting state: AT**

1. A process for the preparation of the peptides corresponding to the general formula:

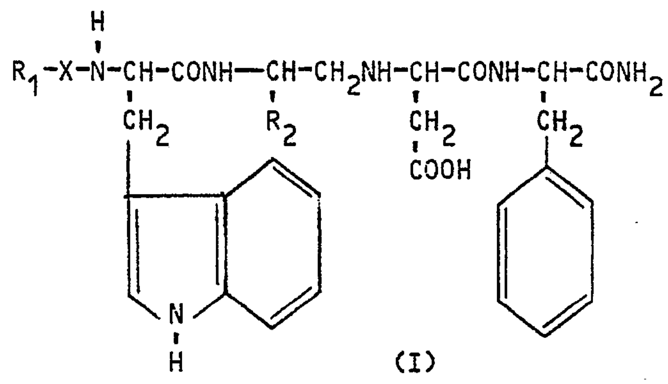

(I)

in which:

– $R_1$ represents hydrogen or a protecting group for the amine group, such as t-butoxycarbonyl, benzyloxycarbonyl or lower alkanoyl groups;

– X represents beta-alanine, glycine or a direct bond between $R_1$ and the amine group;

– $R_2$ represents a group chosen from:

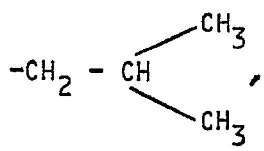 , $-CH_2-CH_2-S-CH_3$ ou $-(CH_2)_3-CH_3$ ;

corresponding to the side chains of the natural amino acids: leucine, methionine and norleucine, and also their salts with inorganic or organic bases, characterized in that an appropriately substituted alpha-amino aldehyde of the formula:

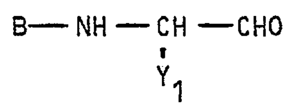

is reacted, in the preseace of a reducing agent, with an alpha-amino acid of the formula:

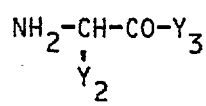

in which formulae: B represents a protecting group for the amine group or a peptide protected on the terminal N amine,

$Y_1$ and $Y_2$ represent the side chains corresponding to the natural alpha-amino acids: leucine, norleucine, methionine and aspartic acid, and

$Y_3$ represents OH or the residue of phenylalaninamide, and that, if appropriate, if B is a protecting group and/or $Y_3$ is OH, the reduced peptide is lengthened by the methods usually employed in peptide chemistry.

2. The process according io claim 1, characterized in that $R_1$ is Boc; X is a direct bond and $R_2$ is such as defined in claim 1.

3. The process according to claim 1, characterized in that X is glycine $R_1$ is Boc and $R_2$ is the side chain of leucine.

4. Use of the peptides obtained according to any one of claims 1 to 3, for the preparation of pharmaceutical compositions containing a peptide of formula I as the active ingredient, in association with a pharmaceutically acceptable vehicle, such as physiological serum.